## Europäisches Patentamt

## European Patent Office

## Office européen des brevets

(11) Veröffentlichungsnummer: **0 182 090**
A2

# EUROPÄISCHE PATENTANMELDUNG

(21) Anmeldenummer: 85113045.0

(22) Anmeldetag: 15.10.85

(51) Int. Cl.⁴: **A 61 F 2/24**

(30) Priorität: 17.11.84 DE 3442088

(43) Veröffentlichungstag der Anmeldung: 28.05.86
Patentblatt 86/22

(84) Benannte Vertragsstaaten: **AT BE CH DE FR GB IT LI NL SE**

(71) Anmelder: **Beiersdorf Aktiengesellschaft,
Unnastrasse 48, D-2000 Hamburg 20 (DE)**

(72) Erfinder: **Pietsch, Hanns, Dr., Mittelweg 25,
D-2000 Hamburg 13 (DE)**
Erfinder: **Kartheus, Holger, Stellinger Weg 19,
D-2000 Hamburg 20 (DE)**
Erfinder: **Reul, Helmut, Dr., Akazienstrasse 65,
D-5160 Düren (DE)**

(54) **Herzklappenprothese.**

(57) Herzklappenprothese mit einem Klappengehäuse in Form eines Ringes und einer innerhalb des Gehäuses gelagerten gewölbten Klappe, die am Klappenring mittels eines flachen, flexiblen Läppchens angelenkt ist, das gegebenenfalls in einem Bereich durch einen Schlitz im Klappenring gezogen ist, die dadurch gekennzeichnet ist, daß die Klappe und der Ring jeweils aus zwei aufeinander bzw. ineinander passenden und fest miteinander verbundenen Hälften bestehen und das Läppchen in Form eines kurzen Bandes mit seinen Endstücken einerseits zwischen den Klappenhälften und andererseits zwischen den Ringhälften «sandwich»-artig eingebettet ist.

EP 0 182 090 A2

I

Beiersdorf Aktiengesellschaft
Hamburg


Herzklappenprothese


Die Erfindung betrifft eine Herzklappenprothese,
d.h. ein prothetisches Verschlußelement zum Ersatz der
Klappen im menschlichen Herzen, insbesondere der
Mitral- und Tricuspidalklappen, mit einem Klappengehäuse in Form eines Ringes und einer innerhalb des
Gehäuses gelagerten Klappe, die zur Anströmrichtung
konvex ausgebildet und mittels eines flachen, flexiblen
Läppchens angelenkt ist, das gegebenenfalls in einem
Bereich durch einen Schlitz im Klappenring gezogen ist.
Der Klappenring weist außerdem vorteilhafterweise eine
kreisringförmige Auflagefläche für die Klappe auf und
wird mit dem üblichen Nahtring am Gewebe befestigt.
Eine vergleichbare Herzklappe ist Gegenstand der DE-PS
28 15 756, auf deren Offenbarungsgehalt im übrigen
verwiesen und bezuggenommen wird.

Die vorliegende Erfindung stellt eine Weiterentwicklung bzw. Verbesserung der in dieser Patentschrift beschriebenen Herzklappe dar.

Ein wesentliches Merkmal der Herzklappe gemäß der
DE-PS 28 15 756 ist das bewegliche Kunststoffgelenk,
welches in der Form eines einstückig mit dem Deckel
(d.h. der Klappe) verbundenen Läppchens eine Lasche
bildet, die durch einen Schlitz im Ring hindurchgezogen
und außen am Ring befestigt wird. An der Stelle, wo
sich das Gelenk befindet, sind der Ring und der Deckel abgeflacht (d.h. die Kreisfläche ist sehnenartig angeschnitten).

Die Hauptbelastung bei der Funktion der Klappe liegt am Kunststoffgelenk, das auf Biegung beansprucht wird. Die Kunststoffe, die zur Ausbildung eines solchen Gelenkes geeignet sind, müssen daher ausgezeichnete Dauerflexibilitätsbeständigkeit und niedrige Shore-Härte aufweisen, um dieser Dauerbelastung zu widerstehen. Es hat sich außerdem in Dauerversuchen gezeigt, daß neben den Biegekräften auch Torsionskräfte auftreten können. Diese Torsionskräfte beanspruchen das Gelenk vom Rand her und können dazu führen, daß das Gelenk von der Seite her einreißt oder daß der Deckel durch den Ring hindurchgedrückt wird.

Es wird in der genannten DE-PS 28 15 756 beschrieben, daß das gelenkbildende Läppchen vorzugsweise einstückig mit dem Deckel verbunden sein soll. Diese "Einstückigkeit" wird dadurch erreicht, daß der Deckel vollständig von beiden Seiten mit Kunststoff beschichtet wird. An der Stelle, wo sich das Gelenk befindet, geht die Beschichtung in Form einer Lasche über den Deckel hinaus. Die Vorteile dieser Herzklappe liegen darin, daß sie eine Gelenkverbindung aufweist, die abgesehen von ihrer Funktionssicherheit keinerlei negative Auswirkungen auf den Blutstrom hat. Mit dieser Konstruktion wird eine weiche Strömungsführung sowohl in der offenen als auch in der geschlossenen Stellung erreicht, und es werden thrombenerzeugende Totwasserzonen, schädliche Reibung sowie blutschädigende hohe Schubspannungen vermieden.

-3-

Durch die Ausbildung des Gelenkes in Form des flachen Läppchens ergeben sich während der Einströmphase im wesentlichen keine Strömungshindernisse, während bei den bereits früher bekannten Herzklappen durch die platzraubende Ausführungsform der Gelenkverbindung eine ziemlich starke Einschränkung des Durchtrittsquerschnittes auftritt. Des weiteren weist das Verschlußelement gemäß der DE-PS 28 15 756 eine relativ niedrige Bauhöhe auf, wobei die Höhe des Klappenringes der Höhe der als Kugelkalotte ausgebildeten Klappe entsprechen kann. Die Gelenkverbindung in Form des flachen Läppchens macht hierbei keine Vergrößerung der Bauhöhe erforderlich, da der Nahtring seitlich vom Gelenk angebracht werden kann.

Die aus der beschriebenen Ausbildung des Gelenkes resultierenden Vorteile werden noch dadurch verstärkt, daß die Klappe als schalenförmiges Element in Form einer Kugelkalotte ausgebildet ist. Die Ausbildung der Klappe als Kugelkalotte dient zum einen der Erhöhung der Festigkeit (im geschlossenen Zustand können Druckdifferenzen bis zu 300 mm Hg auftreten) und hat zum anderen funktionelle Gründe, die nachfolgend am Beispiel der Mitralklappe erläutert werden:

Wenn sich die Herzkammer füllt, öffnet sich die Mitralklappe sehr schnell und der in die Kammer eintretende Blutstrahl trifft die Kammerspitze, breitet sich seitwärts und aufwärts hinter den beiden natürlichen Mitralsegeln aus und erzeugt gleichzeitig einen starken Ringwirbel in der sich ausdehnenden Herzkammer, der die beiden Segel in einer stabilen Position hält. Wenn sich die Einströmung verzögert, verursacht die Druckdifferenz zu beiden Seiten der Segel eine Bewegung in Richtung zur Schließposition. Dadurch ist die Mitralklappe bereits geschlossen, bevor die Kammerkontraktion und damit die Ausströmphase beginnt. Diese Funktionsweise wird durch die Gelenkkonstruktion sichergestellt. Es hat sich gezeigt, daß der hinter der Schale erzeugte Wirbel einen Klappenschluß vor Beginn der Kammerkontraktion bewirkt. Der Durchmesser der Kugelkalotte liegt dabei in der Größenordnung des Wirbeldurchmessers, wodurch sich die Schale harmonisch dem physiologisch vorgegebenen Strömungsverlauf anpaßt.

Die weitere Funktionsweise dieses Klappentyps ist in der deutschen Patentschrift 28 15 756 ausführlich beschrieben.

Nachteile der vorstehend beschriebenen Klappe sind jedoch folgende:

1. Der Kunststoff, der das Läppchen bildet, muß hervorragend auf dem Metalldeckel haften, dies ist für die vorzugsweise verwendeten Polyurethane nicht der Fall. Das als Haftvermittler benutzte Epoxid verbessert zwar die Haftung, diese reicht aber nicht aus, um eine Ablösung der Beschichtung in Dauerfunktion zu verhindern.

-5-

2. Die Beschichtung des Deckels erfolgt vorzugsweise durch Tauchbeschichtung. An den Kanten des Deckels bilden sich dabei dünne Stellen, die besonders am Ansatz der Lasche leicht reißen.

3. Die Verbindung der Lasche mit dem Ring erfolgt über fest angezogene Fäden, die in der äußeren Nut des Ringes verlaufen, oder durch Verklebung in dieser rinnenförmigen Ausnehmung. Die Festigkeit dieser Verbindung ist jedoch nicht ausreichend, wie sich im Dauerbetrieb herausgestellt hat. Der Deckel kann sich lockern und sich sodann im Ring querstellen oder gänzlich herausgezogen werden.

4. Der kalottenförmige Deckel weist an seiner angeschnittenen Seite zum Gelenk hin einen bogenförmigen Verlauf auf, so daß das gelenkbildende Läppchen in seiner Querrichtung ebenfalls gewölbt ist. Dies führt zu einer erhöhten Belastung an seinen Rändern und damit leicht zu Kerbrissen vom Rande her.

Aufgabe der Erfindung war es daher, die hervor- ragenden strömungstechnischen Eigenschaften der Herz- klappe als solcher zu erhalten, jedoch die geschilderten Nachteile zu beseitigen.

Diese Aufgabe wird erfindungsgemäß gelöst durch eine Herzklappenprothese mit einem Klappengehäuse in

- 6 -

Form eines Ringes und einer innerhalb des Gehäuses gelagerten gewölbten Klappe, die am Klappenring mittels eines flachen, flexiblen Läppchens angelenkt ist, das gegebenenfalls in einem Bereich durch einen Schlitz im Klappenring gezogen ist, die dadurch gekennzeichnet ist, daß die Klappe und der Ring jeweils aus zwei aufeinander bzw. ineinander passenden und fest miteinander verbundenen Hälften bestehen und das Läppchen in Form eines kurzen Bandes mit seinen Endstücken einerseits zwischen den Klappenhälften und andererseits zwischen den Ringhälften "sandwich"-artig eingebettet ist.

Die gelenkbildende Lasche ist also ebenfalls einstückig mit dem Deckel verbunden, jedoch nicht wie bei der Herzklappe gemäß DE-PS 28 15 756 von außen, sondern durch Einlegen eines Bandes aus textilem Material oder vorzugsweise aus flexiblem Kunststoff zwischen zwei Deckelhälften in Form eines "Sandwiches".

Bei der Herstellung der erfindungsgemäßen Herzklappe wird die gelenkbildende Lasche so mit ihrem einen Ende zwischen die beiden Deckelhälften gelegt, daß an der abgeflachten Seite des Deckels ca. 5-10 mm des Bandes herausragen und sich ca. 60-80% seiner Länge im Deckel befinden. Die beiden Deckelteile werden dann mit dem dazwischen eingelegten Band verbunden, entweder durch Verschweißen, Verkleben, Verschrauben oder Vernieten, vorzugsweise durch Verschweißen mit Ultraschall. Der Deckel wird mit dem Ring dadurch verbunden, daß das aus der abgeflachten Seite des Deckels herausragende Band vorzugsweise durch einen kleinen Schlitz im inneren Teil des Ringes hindurch gezogen wird bis der Deckel kein Spiel mehr aufweist, der äußere Teil des Ringes darüber geschoben wird und dann beide Teile des Ringes miteinander vorzugsweise durch Verschweißen verbunden werden. Das Bändchenende kann auch direkt zwischen die beiden Ringhälften gelegt und eingeschweißt werden. Auf diese Weise ist das Bändchen, welches das Gelenk bildet, fest und dauerhaft mit seinen beiden Endstücken im Deckel und im Ring verankert.

Die Herstellung der Ring- und Deckelhälften erfolgt vorzugsweise durch Spritzguß. Als Materialien dafür sind thermoplastische Kunststoffe geeignet, die nichttoxisch, nicht enzymatisch-abbaubar und blutverträglich sind. An mechanischen Eigenschaften werden von dem Werkstoff hohe Steifigkeit sowie gute Bruch- und Biegefestigkeit verlangt. Auch soll das Material hydrolysebeständig sein und keine toxischen Hilfsstoffe enthalten.

Stoffe, die diese Anforderungen erfüllen sind beispielsweise hochmolekulare Niederdruckpolyethylene aus dem Ziegler-Natta-Verfahren. Hervorragend geeignet sind außerdem Polycarbonat, Polyoxymethylen und Polyethersulfon, wobei ersteres besonders bevorzugt ist.

Eine weitere besondere Ausführungsform der Herzklappe besteht darin, daß die flexible Lasche an den inneren Ringteil angesetzt ist, d.h. einstückig mit diesem ausgebildet ist, was beispielsweise durch

-8-

Anformung gleich bei der Herstellung erreicht werden kann, und mit seinem freien Ende zwischen den beiden Klappenhälften "sandwich"-artig eingebettet ist.

In diesem Fall besteht der innere Teil des Ringes nicht aus den vorstehend genannten thermoplastischen Kunststoffen, sondern aus dem flexiblen Material der Lasche.

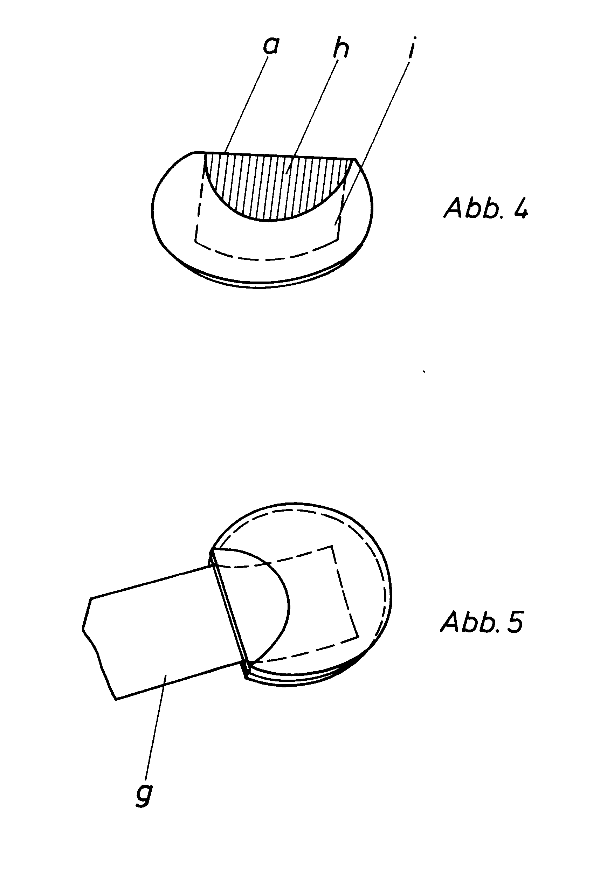

Zur Entlastung des Gelenkes im Sinne der vorstehend unter Punkt 4 aufgeführten Nachteile wurde die Konstruktion des Deckels in einer besonders bevorzugten Ausführungsform gegenüber der DE-PS 28 15 756 dahingehend geändert, daß der gewölbte Deckel zum Gelenk hin abgeflacht ist, so daß das gelenkbildende Läppchen in der Drehachse eben liegt. Wie aus Abbildung 4 ersichtlich, ist diese Abflachung vorteilhafterweise so ausgestaltet, daß ihre Umfangslinie über der Gelenkachse als Basis etwa einen Halbkreis bildet.

Die Lasche bzw. das Gelenk sind der kritische Teil der Herzklappenprothese. Die Anforderungen an das Material sind: hervorragende biologische Verträglichkeit, keine Toxizität und auch keine toxischen eluierbaren Bestandteile, Hydrolysebeständigkeit, keine enzymatische Abbaubarkeit, ausgezeichnete Flexibilität, extrem hohe Dauerwechselbiegefestigkeit mit einer Shore-Härte A von 20 bis 70 und kautschukelastische Eigenschaften bei 37°C und darunter.

-9-

Stoffe mit kautschukelastischen Eigenschaften bei 37$^\circ$C oder darunter nennt man auch Elastomere. Dies sind (nach ELIAS in "Makromolekule", Hüthig und Wepf Verlag Basel, Heidelberg 1971, S. 22) makromolekulare Substanzen mit Elastizitätsmoduln von $10^6 - 10^7$ dyn/cm$^2$ und einer reversiblen Dehnbarkeit von 200 bis 1000% (vgl. auch B. Vollmert, Grundriss der makromolekularen Chemie, Springer Verlag, Berlin, Göttingen, Heidelberg, 1962, S. 397 ff, wo die molekularen Vorgänge der elastischen Verformung ausführlich und anschaulich beschrieben sind).

Von den üblichen Elastomeren sind für die hier beschriebene Verwendung besonders die segmentierten Polyetherurethane, Polyetherharnstoffe, Polydimethylsiloxane, Mischpolymere aus Ethylen und Propylen (EPM), Mischpolymere aus Ethylen, Propylen und einem Dien (EPDM) oder ein Vinylidenfluorid-hexafluorpropylen-Mischpolymerisat (z.B. Viton A$^®$, Du Pont) geeignet. Besonders bewährt haben sich Polydimethylsiloxan und EPDM. Gemeinsam ist allen diesen Elastomeren eine Glastemperatur unter 0$^\circ$C (Definition dieses Begriffes beispielsweise in J. Brandrup, E. H. Immergut, Polymer Handbook, Interscience Publishers, New York, London, Sydney, 1966, III, 61 ff).

Wie bereits erwähnt, ist die Lasche beim Einsatz der Herzklappen einer hohen Wechselbiege- und Torsionsbelastung ausgesetzt. Besonders letztere birgt dabei die Gefahr eines seitlichen Einreißens der Lasche in sich. Ihre Kanten müssen deshalb frei von Quetschungen und Unregelmäßigkeiten sein, wie sie beispielsweise durch

-10-

Schneiden mit einem Messer, Skalpell oder einer Schere entstehen können. Vorteilhafterweise sind die seitlichen Kanten der Lasche daher abgerundet, vorzugsweise sogar noch etwas verdickt im Vergleich zur sonstigen Dicke des Bändchens. Die gerundeten Kanten werden entweder bei Verwendung von thermoplastischen Elastomeren durch Schmelzschnitt (Hitzdraht- oder Laserstrahlschnitt) oder bei Verwendung von Siliconkautschuken durch entsprechende Formgebung erzeugt.

Die Dicke des gelenkbildenden Läppchens beträgt 0,1-2,0 mm, vorzugsweise 0,3-0,8 mm.

Nachstehend wird die erfindungsgemäße Herzklappenprothese anhand der Abbildungen beispielsweise erläutert:

Abbildung 1 zeigt den Ring in der Aufsicht mit dem sehnenartigen Anschnitt a. Dieser bildet die Gelenkachse.

Abbildung 2 zeigt den Ring im Querschnitt bestehend aus einem Ring-Außenteil d und einem Ring-Innenteil b. Der Ring-Innenteil weist an der abgeflachten Seite a einen Schlitz c auf.

Beim Zusammensetzen der Klappe wird durch diesen Schlitz die gelenkbildende Lasche g (siehe Abb. 5) hindurchgezogen und sodann der Ring-Außenteil über den Ring-Innenteil geschoben. Die aus dem Schlitz heraustretende Lasche wird dabei zwischen Ring-Außenteil und Ring-Innenteil eingeklemmt. Ring-Außenteil und

-11-

Ring-Innenteil mit eingeklemmter Lasche werden sodann miteinander verklebt und/oder verschweißt und der überstehende Teil der Lasche kurz abgeschnitten.

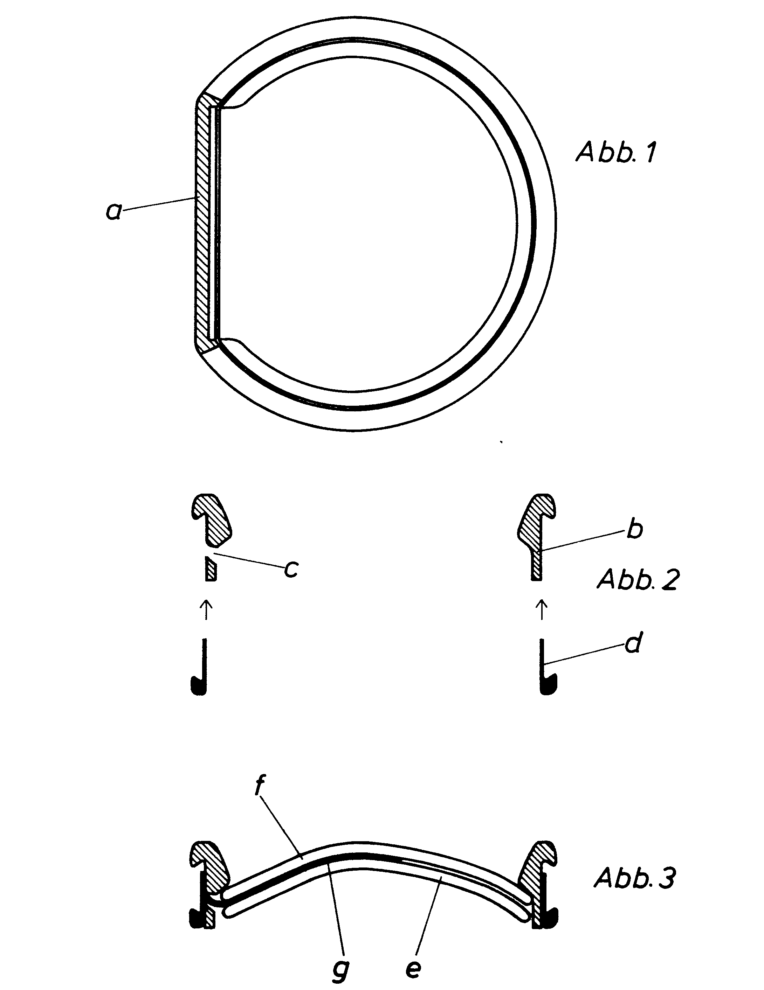

Abbildung 3 zeigt die vollständige Klappe ohne Nahtring im Querschnitt, nämlich das Deckeloberteil f, Deckelunterteil e und dazwischen die gelenkbildende Lasche g, die durch die beiden Ringteile b und d an der Stelle des Schlitzes c eingeklemmt ist.

Abbildung 4 zeigt eine Deckelhalbschale e bzw. f mit dem sehnenförmigen Anschnitt a und der kreisbogenförmigen Abflachung h und einer Aussparung i für die einzulegende Gelenklasche.

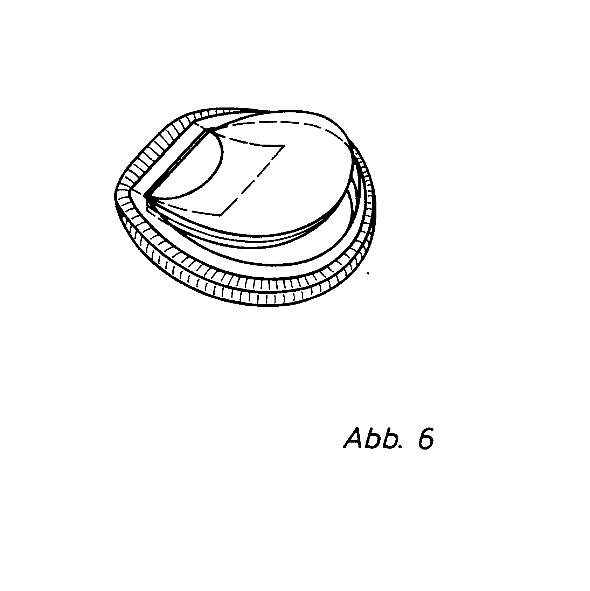

Abbildung 5 zeigt die beiden zusammengefügten Deckelhälften e und f mit eingelegter Gelenklasche von oben gesehen entsprechend Abbildung 6.

Abbildung 6 zeigt eine fertige Herzklappenprothese mit textilem Nahtring im geöffneten Zustand.

0182090

-12-

Patentansprüche

1) Herzklappenprothese mit einem Klappengehäuse in Form eines Ringes und einer innerhalb des Gehäuses gelagerten gewölbten Klappe, die am Klappenring mittels eines flachen, flexiblen Läppchens angelenkt ist, das gegebenenfalls in einem Bereich durch einen Schlitz im Klappenring gezogen ist, dadurch gekennzeichnet, daß die Klappe und der Ring jeweils aus zwei aufeinander bzw. ineinander passenden und fest miteinander verbundenen Hälften bestehen und das Läppchen in Form eines kurzen Bandes mit seinen Endstücken einerseits zwischen den Klappenhälften und andererseits zwischen den Ringhälften "sandwich"-artig eingebettet ist.

2) Herzklappenprothese mit einem Klappengehäuse in Form eines Ringes und einer innerhalb des Gehäuses gelagerten gewölbten Klappe, die am Klappenring mittels eines flachen, flexiblen Läppchens angelenkt ist, dadurch gekennzeichnet, daß die Klappe und der Ring jeweils aus zwei aufeinander bzw. ineinander passenden und miteinander festverbundenen Hälften bestehen, das Läppchen einstückig mit der inneren Ringhälfte ausgebildet und mit seinem freien Ende zwischen den beiden Klappenhälften "sandwich"-artig eingebettet ist.

3) Herzklappenprothese gemäß Anspruch 1 oder 2, dadurch gekennzeichnet, daß die gewölbte Klappe zum Gelenk hin abgeflacht ist.

-13-

4) Herzklappenprothese gemäß Anspruch 1, 2 oder 3, dadurch gekennzeichnet, daß das gelenkbildende Läppchen eine Dicke von 0,1 bis 2,0 mm und an den Seitenrändern runde Konturen aufweist.

5) Herzklappenprothese gemäß einem oder mehreren der Ansprüche 1-4, dadurch gekennzeichnet, daß das gelenkbildende Läppchen oder das Läppchen und die mit ihm einstückig ausgebildete innere Ringhälfte aus kautschuk-elastischen Polyetherurethanen, Polyetherharnstoffen, Polyetherurethan/Siliconhybrid-Elastomeren, Ethylen/Propylen-Mischpolymeren (EPM), Ethylen/Propylen/Dien-Mischpolymeren (EPDM) oder Polydimethylsiloxanen besteht.

6) Herzklappenprothese gemäß Anspruch 5, dadurch gekennzeichnet, daß das gelenkbildende Läppchen oder das Läppchen und die mit ihm einstückig ausgebildete innere Ringhälfte aus Polydimethylsiloxan (Silikonkautschuk) oder einem Mischpolymeren aus Ethylen, Propylen und einem Dien (EPDM) besteht.

7) Herzklappenprothese gemäß einem oder mehreren der Ansprüche 1-6, dadurch gekennzeichnet, daß die Ring- und Klappenhälften bzw. die äußere Ringhälfte und die Klappenhälften aus einem thermoplastischen Kunststoff wie Niederdruckpolyethylen, Polycarbonat, Polyoxymethylen oder Polyethersulfon bestehen und vorzugsweise im Spritzgußverfahren hergestellt

0182090

-14-

worden sind.

8) Herzklappenprothese gemäß einem oder mehreren der Ansprüche 1-7, dadurch gekennzeichnet, daß die Klappen- und Ringhälften jeweils miteinander und mit dem Läppchen verklebt oder verschweißt sind.

9) Herzklappenprothese gemäß Anspruch 8, dadurch gekennzeichnet, daß die Teile durch Ultraschall verschweißt sind.

*Abb. 1*

*a*

*c*

*b*

*Abb. 2*

*d*

*f*

*Abb. 3*

*g*    *e*

Abb. 4

Abb. 5

0182090

*Abb. 6*